# EUROPEAN PATENT APPLICATION

(11) **EP 4 846 988 A1**
(43) Date of publication of application: **02.12.2026**
(21) Application number: 26180398.5
(22) Date of filing: 21.05.2026
(51) Int. Cl.: A61F 9/009, A61B 3/00, A61B 3/10, A61B 3/12

(54) **APPARATUS AND SYSTEM FOR SAFETY INTERLOCK FOR LASER APPLICATIONS**

(30) Priority: 27.05.2025 US 202563812790 P
(71) Applicant: Phoenix-Micron, Inc., Bend, Oregon 97703 (US)
(72) Inventor: THORN, Jonathan Roy, Bend, 97703 (US)
(74) Representative: Diehl & Partner

(57) **Abstract**

The present disclosure provides an apparatus, system, and method for safety interlock for laser applications. The apparatus comprises a housing including a laser and a lens assembly. The apparatus further includes a lens tip disposed on the lens assembly configured to make electrical and optical contact with a subject. A portion of the lens tip is conductive. The apparatus further includes an open circuit between the lens tip and the laser. The laser is capable of being emitted when a conductive member is in contact with the lens tip to close the open circuit and is prevented from being emitted when the conductive member is not in contact with the lens tip.

## Description

### CROSS-REFERENCES TO RELATED APPLICATIONS

The present application claims the benefit and priority of U.S. Provisional Application No. 63/812,790, filed May 27, 2025, entitled "Apparatus And System For Safety Interlock for Laser Applications," the entire content of which is incorporated herein by reference for all purposes.

### BACKGROUND OF THE INVENTION

Eye-imaging devices are used to detect potential eye diseases, vision impairments, and indicators of various other diseases. Eye-imaging devices may include multiple lenses and other optical elements. These eye-imaging devices may use various imaging modalities, such as optical coherence tomography, laser injection, and electroretinogram techniques. For laser injection, the eye-imaging device may include a laser that is emitted onto a particular region of the eye of a specimen. The laser may be used to treat eye-related conditions such as correct refractive errors like nearsightedness and farsightedness, treat eye diseases like diabetic retinopathy and glaucoma, and assist in cataract or tissue removal.

In conventional systems that emit lasers for eye-related procedures, there is a gap between the laser and the eye of the subject. Because the laser is used to treat eye conditions (e.g., ablate tissue), a safety interlock is needed to prevent the laser from inadvertently being fired and causing damage to the subject, medical personnel, or others. Conventionally, the safety interlock is linked to the door to the operating room in which the laser is administered, and the safety interlock prevents the laser from being fired when the door is open. However, even when the door to the operating room is closed, the laser may move through air and potentially cause damage to personnel.

Although eye-imaging devices have been developed, there is a need in the art for improved methods and systems related to safety interlocks for lasers of eye-imaging devices.

### SUMMARY OF THE INVENTION

The present disclosure relates to an apparatus, system, and method for providing a safety interlock for laser applications. In particular, the present disclosure provides an apparatus, system, and method for establishing safety interlock for an eye-imaging device that utilizes an open circuit between a laser and a lens tip that can be closed by contacting a conductive member with the lens tip. The conductive member can be an eyeball of a subject that closes the circuit such that a laser can be emitted. In some embodiments, the conductive member can be an intermediary between the eye and the conductive member (e.g., a gel or saline solution). Beneficially, the laser is prevented from being emitted unless the conductive member (e.g., eyeball) is in contact with the lens tip, which provides the safety interlock for the apparatus. As such, the laser is not emitted through the air, which reduces the need for conventional safety interlocks.

Embodiments of the present invention provide an apparatus comprising: a housing comprising a laser and a lens assembly; a lens tip disposed on the lens assembly configured to contact an eye of a subject, wherein a portion of the lens tip is conductive; and an open circuit between the lens tip and the laser; wherein the laser is configured to emit when a conductive member is in contact with the lens tip to close the open circuit.

Embodiments of the present invention provide a method comprising: providing an apparatus comprising: a housing comprising a laser and a lens assembly; a lens tip disposed on the lens assembly configured to contact an eye of a subject, wherein a portion of the lens tip is conductive; and an open circuit between the lens tip and the laser; wherein the laser is configured to emit when a conductive member is in contact with the lens tip to close the open circuit; grounding the subject with an electrical ground; contacting the eye of the subject with the lens tip; and emitting the laser in response to contacting the eye with the lens tip based on an electrical path established between the lens tip, the subject, and the electrical ground.

Embodiments of the present invention provide a system for applying a laser, the system comprising: an apparatus housing comprising a laser; a camera disposed in the apparatus housing and comprising a lens assembly; a lens tip disposed on the lens assembly configured to contact a conductive member of a subject, wherein a portion of the lens tip is conductive; and an electrical ground in contact with the subject, wherein the lens tip, the subject, and the electrical ground comprise an electrical path when the conductive member of the subject is in contact with the lens tip, and wherein the laser is configured to be emitted based on the electrical path.

### DETAILED DESCRIPTION OF SPECIFIC EMBODIMENTS

FIG. 1 illustrates a cross-sectional view of an eye-imaging device that emits a laser.
FIG. 2 illustrates a cross-sectional view of an eye-imaging device that emits a laser.
FIG. 3 illustrates a perspective view of a lens assembly including a lens tip according to some embodiments.
FIG. 4 illustrates a view of an open circuit for a safety interlock for an eye-imaging device that emits a laser.
FIG. 5 illustrates a schematic diagram of an open circuit for a safety interlock for an eye-imaging device that emits a laser.
FIG. 6 illustrates a view of a closed circuit for a safety interlock for an eye-imaging device that emits a laser.
FIG. 7 illustrates a schematic diagram of a closed circuit for a safety interlock for an eye-imaging device that emits a laser.
FIG. 8 illustrates a schematic diagram of a closed circuit with an amplifier for a safety interlock for an eye-imaging device that emits a laser.
FIG. 9 illustrates a diagram of a circuit for a safety interlock for an eye-imaging device that emits a laser.
FIG. 10 illustrates an apparatus for positioning a specimen for imaging.
FIG. 11 illustrates a view of an apparatus including grounding components.

### DETAILED DESCRIPTION OF SPECIFIC EMBODIMENTS

The present disclosure describes a number of embodiments related to an apparatus, system, and method for a safety interlock for laser applications from an eye-imaging device. In some embodiments, the apparatus includes a housing including a laser and a lens assembly. A lens tip is disposed on the lens assembly and is configured to contact an eye of a subject. At least a portion of the lens tip can be conductive. There is an open circuit between the lens tip and the laser, so the laser is prevented from being emitted unless a conductive member is in contact with the lens tip (i.e., the conductive portion of the lens tip) to close the open circuit. The conductive member can be the eyeball of the subject. In this embodiment, when the eyeball of the subject is in contact with the lens tip to close the open circuit, the laser can be emitted. If the eyeball of the subject is removed from contacting the lens tip, then the circuit is opened, and the laser is terminated. Additionally, other conductive members can be used to close the open circuit. For example, another conductive surface coupled to the eye-imaging device can be used. This provides a safety interlock such that the laser cannot be emitted in an unsafe condition (e.g., when the subject is not in contact with the lens tip). In some embodiments, the conductive member can be located at another region of the eye imaging device or can be separate from the eye imaging device. For example, a separate device may include an element that requires the user to contact (e.g., using the hand) the element to complete the circuit. In some embodiments, the conductive member may be skin or another part of the subject other than the eyeball, or can be used in combination with the eyeball to complete the circuit. Additionally, in some embodiments, the laser can be applied to skin or an area other than the eye.

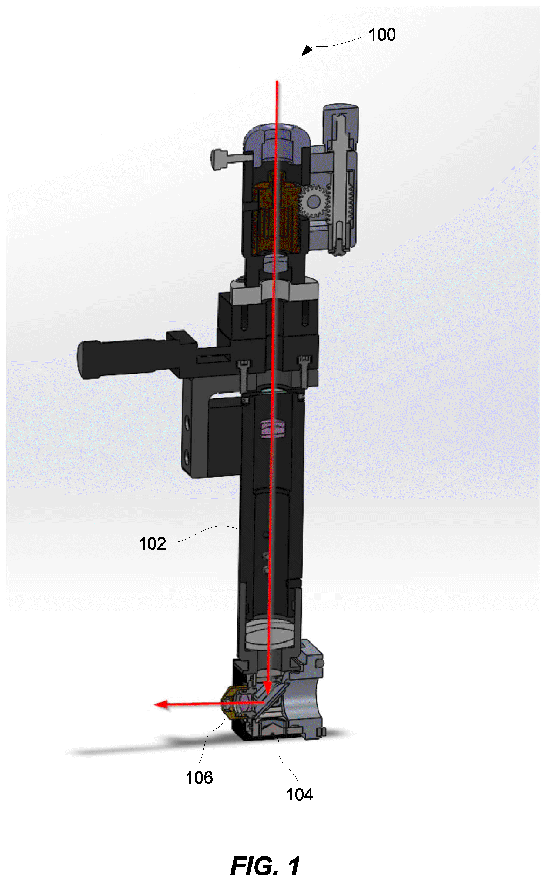

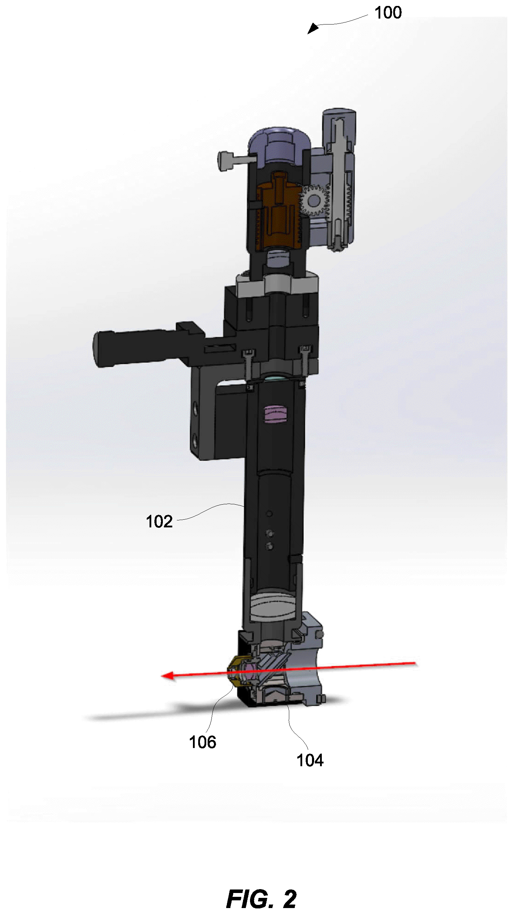

FIGS. 1-2 illustrate cross-sectional views of an eye-imaging device 100 that emits a laser. The eye-imaging device 100 includes a laser injector that emits the laser. The eye-imaging device includes a housing 102 with the laser and a lens assembly 104. A laser beam emitted by the laser injector takes the path illustrated by the arrows in FIG. 1. For example, an initial laser beam, originating from a laser source, first encounters focusing optics. These optics, which could be one or more lenses, are configured to converge the laser beam to a smaller spot. This initial focusing can help to increase the intensity of the laser beam. Next, the focused laser beam can be provided to a beamsplitter. A beamsplitter is an optical device that partially transmits and partially reflects an incident light beam. For example, the beamsplitter is positioned to reflect a portion (or all) of the laser beam along a desired path. The remaining portion of the beam can pass through the beamsplitter. The reflected laser beam travels towards and exits through a lens tip of the lens assembly. This lens tip is configured to focus or shape the laser beam before it exits the lens assembly. That is, the laser beam passes through laser focusing optics, bounces off a laser beamsplitter, and exits through a lens tip 106 that is disposed on the lens assembly 104. The arrow in FIG. 2 illustrates the direction in which imaging and illumination happen for the eye-imaging device. After the laser is emitted, the eye imaging camera can work in conjunction with the laser to achieve illumination and/or imaging of the eye structures.

In some embodiments, the laser itself can be a controlled illumination source. For example, in Scanning Laser Ophthalmology (SLO) and Optical Coherence Tomography (OCT) applications, the laser beam is scanned across the retina in a specific pattern. Different wavelengths of the laser can be selected to interact with specific tissue layers of the eye structures. For example, red or near-infrared lasers can penetrate deeper into the choroid, green lasers are often used to enhance the contrast of blood vessels and the retinal nerve fiber layer, and blue lasers can be used for fundus autofluorescence imaging, where they excite naturally fluorescent molecules in the retina. As the laser light interacts with the various structures of the eye (retina, cornea, lens, etc.), a portion of the light is scattered back towards the eye-imaging device. In some techniques like fundus autofluorescence, the laser light excites fluorescent molecules, which then emit light at a different wavelength. Specialized lenses and mirrors (optics) within the eye imaging camera gather this backscattered or emitted light. The collected light (either the backscattered laser light in SLO or the interference signal in OCT) is focused onto a detector, such as a photomultiplier tube or a CCD/CMOS sensor. The detector measures the intensity of the light at each point of the scanned area or the interference pattern. The signals from the detector are then processed by a computer to reconstruct a digital image of the eye structure being examined. This image can be a surface view (like in SLO) or a cross-sectional view (like in OCT).

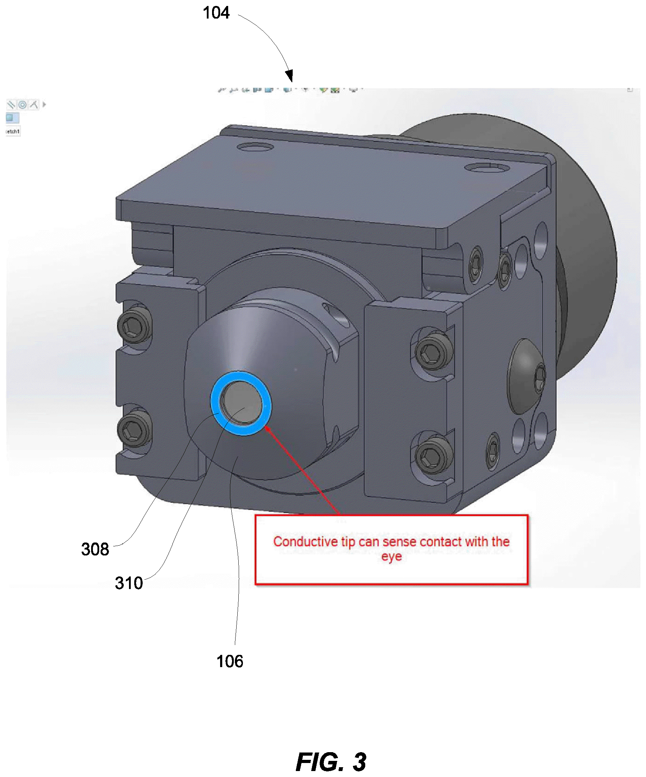

FIG. 3 illustrates a view of a lens tip 106 according to some embodiments. The lens tip 106 is disposed on a lens assembly 104. The lens tip 106 can guide the exiting laser. For example, the lens tip 104 can focus the laser beam, collimate the laser beam, or diverge the laser beam depending on the specific application. The lens tip 106 is configured to contact an eye of a subject. The lens tip 106 is the exit point of the emitted laser beam. In some embodiments, the lens tip 106 is the distal portion of the lens assembly 104. At least a portion of the lens tip 106 is conductive. For instance, the lens tip 106 can include a contact region 308 that can contact the eye of the subject. The contact region 308 can be conductive (e.g., electrically conductive, ionically conductive, or thermally conductive). To be electrically conductive, the contact region 308 can be made from a material with sufficient electrical conductivity. Examples of electrically conductive materials may be metals such as copper, silver, and gold. For ionic conductivity, the contact region 308 can be made from a material with sufficient ionic conductivity. Examples of ionically conductive materials can include sodium beta-alumina, complex hydrides, and sulfide-based materials. For thermal conductivity, the contact region 308 can be made from a material with sufficient ability to transfer heat. Examples of thermally conductive materials can include metals such as silver, copper, gold, aluminum, and diamond, as well as some ceramics such as aluminum nitride. The contact region 308 may partially surround a portion of a lens 310 of the lens assembly 104. In some embodiments, the contact region 308 may completely surround the lens 310 of the lens assembly 104, as shown in FIG. 3. In some instances, a surface region of the lens tip 106 can be conductive. In some embodiments, the lens tip 106 can be conical and include the contact region 308.

In some embodiments, the laser is in communication with a conductive portion (e.g., the contact region 308) of the lens tip 106. That is, a controller of the laser or circuitry controlling an output of the laser can be in communication with the conductive portion. So, the laser may be prevented from being emitted unless a conductive member is in contact with the lens tip 106 to close an open circuit between the lens tip 106 and the laser. The conductive member can be an eyeball of the subject that contacts the conductive portion of the lens tip 106. An electrical ground can also be in contact with the subject. As such, the lens tip 106, the subject, and the electrical ground form an electrical path when the open circuit is closed. Alternatively, the conductive member may be a gel on the eye of the subject that provides an optical coupling between the imaging lens and the cornea of the eye. In some embodiments, the gel can include a saline solution. In the case of a saline solution, contact with the saline solution on the eye can also complete the electrical circuit.

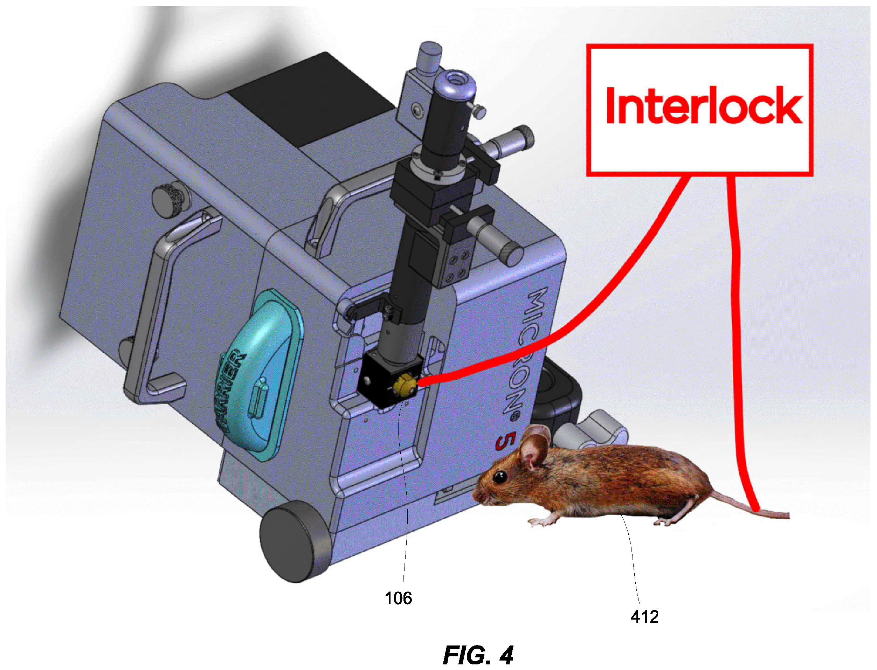

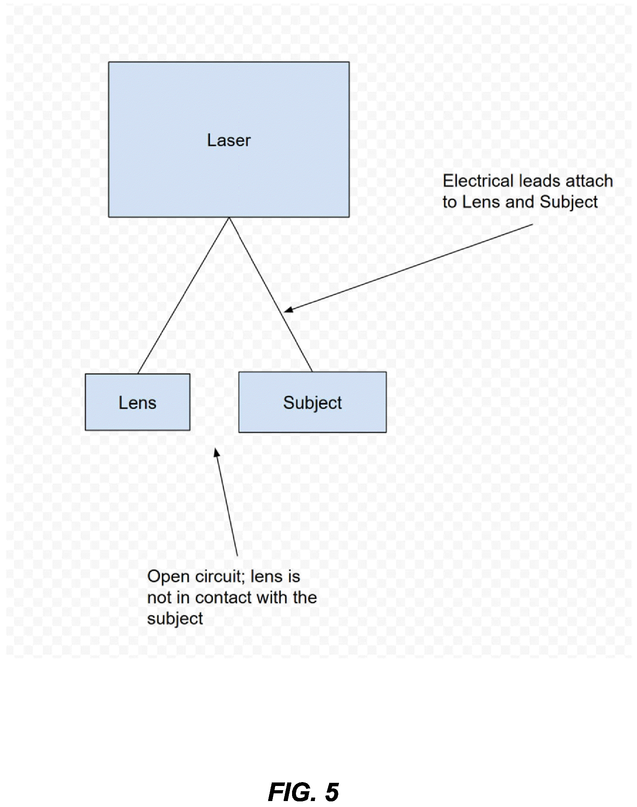

FIGS. 4-5 illustrate open circuits for a safety interlock for a laser. Conductive leads can be attached to the lens tip 106 and to a subject 412. The conductive leads attached to the lens tip 106 can be attached to the conductive portion of the lens tip 106. The conductive leads attached to the subject 412 can be an electrical ground. In some embodiments, electrical ground may be a probe attached to the subject 412, a clip attached to the subject 412, a conductive gel in contact with the subject 412, or a mechanism for positioning the subject 412 for imaging. As shown in FIGS. 4-5, when the lens tip 106 is not in contact with a conductive member, such as an eye of the subject 412, there is an open circuit between the laser and the lens tip 106. Accordingly, the laser is not emitted.

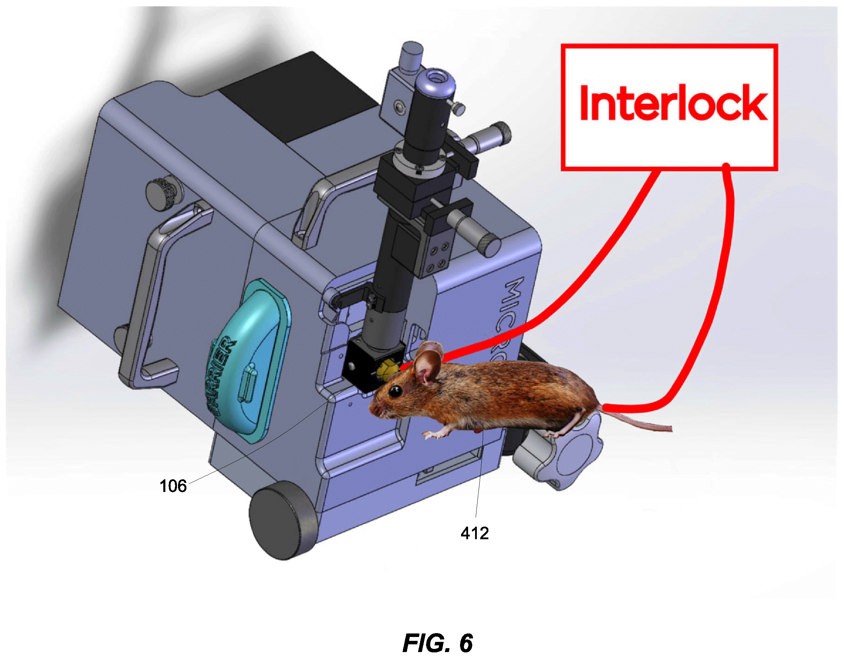

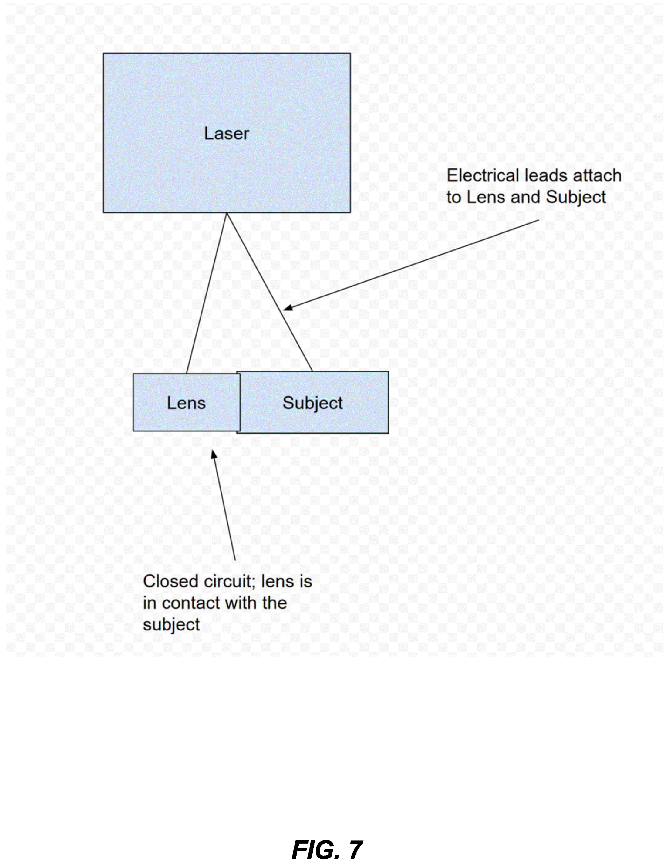

FIGS. 6-8 illustrate closed circuits for a safety interlock for a laser. Similarly to FIGS. 4-5, conductive leads can be attached to the lens tip 106 and to the subject 412. The open circuit prevents an operator from emitting a laser from the laser assembly. The open circuit can be closed by contacting the conductive portion of the lens tip 106. With the lens tip 106 in contact with the conductive member of the subject 412, the open circuit between the laser and the lens tip 106 is closed. When the circuit is closed, an operator can emit the laser. The laser may be emitted automatically when the circuit is closed or based on an action performed by the operator (e.g., a button push). In addition, there is an electrical path between the lens tip 106, the subject 412, and the electrical ground when the open circuit is closed. There can also be a visual indicator that indicates a status of the electrical path. For example, the visual indicator may be a light emitting diode (LED) that emits a first color (e.g., red) when the circuit is open and the electrical path is not established and that emits a second color (e.g., green) when the open circuit is closed, the electrical path is established, and/or the laser is being emitted.

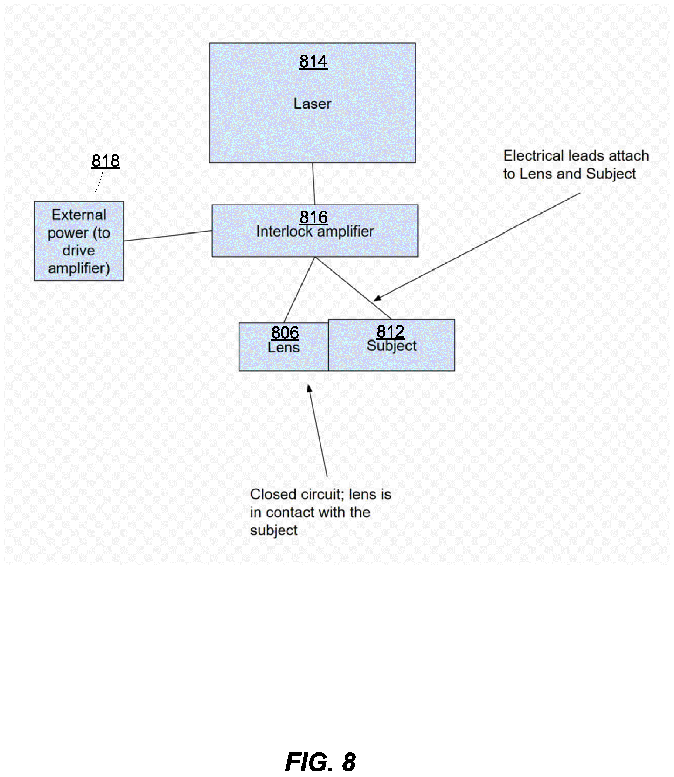

As shown in FIG. 8, some embodiments may include an amplifier circuit 816 disposed between the laser 814 and the lens tip 806. The amplifier circuit 816 is powered by an external power source 818. The electrical path can further include the amplifier circuit 816 when the open circuit is closed. The amplifier circuit 816 can be a high gain amplifier or other circuitry that can be used due to the high impedance of the subject 812.

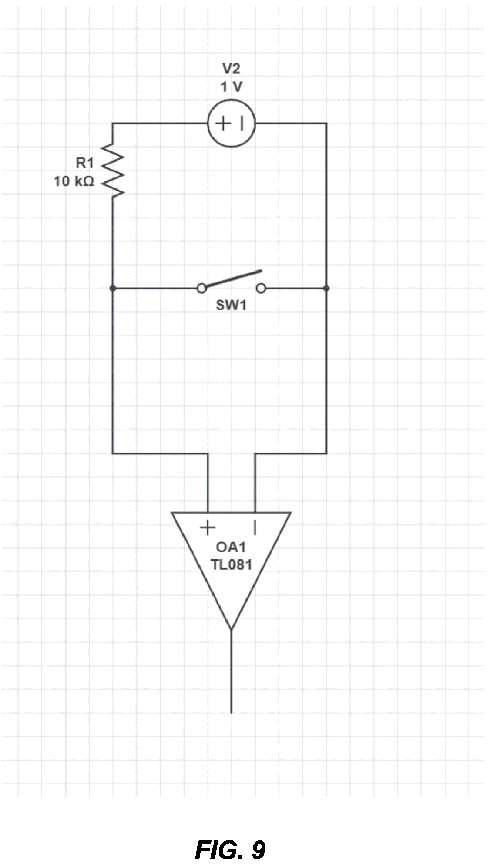

FIG. 9 illustrates a diagram of a circuit for a safety interlock for a laser. The circuit includes an amplifier circuit, as described in FIG. 8, but other embodiments may not include the amplifier circuit. A subject (e.g., subject 412 in FIG. 4) can be the switch (SW1). So, when a conductive member of the subject is in contact with a lens tip, an open circuit between the lens tip and a laser is closed and an electrical path between the lens tip, the subject, and an electrical ground is established. As a result, the laser can be emitted. When the conductive member of the subject is removed from contacting the lens tip, the open circuit is reestablished, so the laser is terminated because the electrical path is removed.

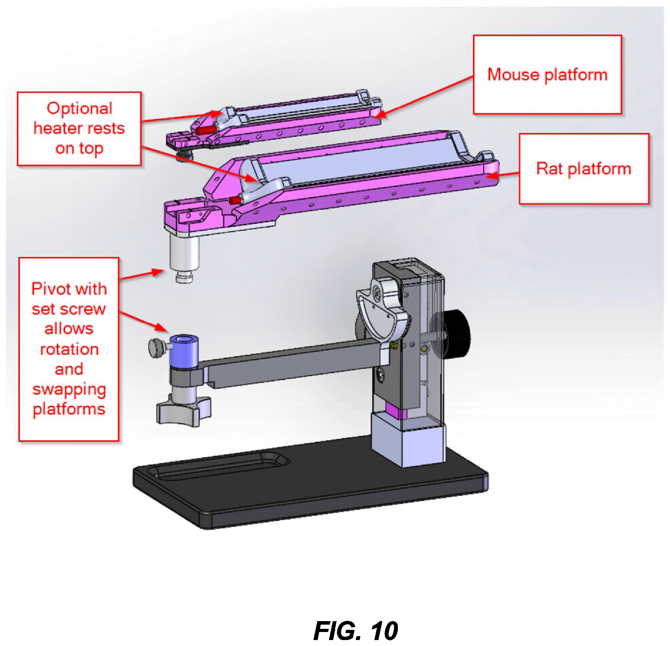

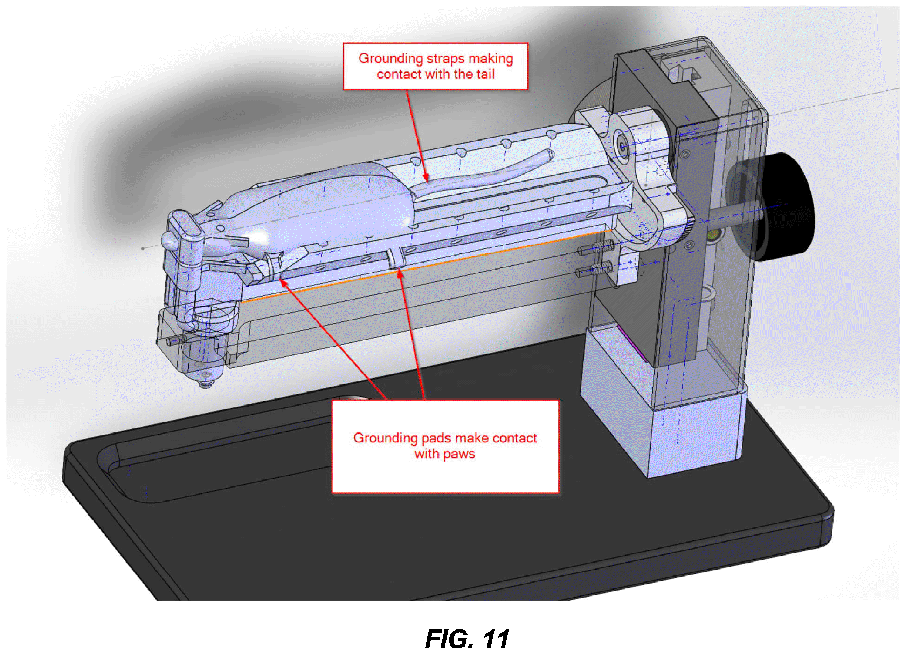

In some embodiments, the electrical ground is a mechanism (e.g., a stand) for receiving and positioning a specimen, as illustrated in FIGS. 10-11. The stand has at least six degrees of freedom of movement in three-dimensional space. For example, the stand is free to move positions in a forward/backward direction, up/down direction, and left/right direction in three perpendicular axes, combined with changes in orientation through rotation about three perpendicular axes, often termed yaw (normal axis), pitch (transverse axis), and roll (longitudinal axis). The stand includes a base member and an upright member. The base member is configured to contact or rest on a surface (e.g., a table surface). The upright member extends from the base member. In some embodiments, the upright member is perpendicular to the base member. The mechanism provides a low-profile stand that incorporates multiple functionalities without occluding the imaging path of an imaging device. For example, the stand may include a heating element that can maintain the body temperature of a specimen. The mechanism may include integrated circuity for sensors to detect various features of the specimen, including blood oxygen, heart rate, blood pressure, respiration rate, or combinations thereof. Additionally, the mechanism provides an integrated cable and hose management system that provides multiple functionalities to the mechanism without occluding the imaging path of an imaging device or restricting movement of the stand.

The mechanism includes an adjustable arm mounted to the upright member. The adjustable arm includes a mounting arm having a first end and a second end. The first end of the mounting arm is attached to the upright member. In some embodiments, the first end of the mounting arm is attached to a rotatable support disposed on the upright member. The rotatable support disposed on the upright member can be coupled to a first adjustment means. For example, the first adjustment means can be a knob, a button, a switch, a handle, or other means for exerting a force on the rotatable support. The first adjustment means is configured to adjust the tilt (e.g., yaw) of the adjustable arm via the rotatable support. For example, the rotatable support may include a first set of grooves that mate with a second set of grooves disposed on the mounting arm such that when the first adjustment means is actuated, the adjustable arm is tilted in a direction.

The adjustable arm includes a pivot attached to the mounting arm. In some embodiments, the pivot is disposed at a distal end of the mounting arm. The pivot connects the mounting arm to a stand. For example, the pivot is coupled to both the mounting arm and the stand. The stand may be positioned over the mounting arm. The pivot is configured to allow the stand to move about a 360° axis. In some embodiments, the pivot includes a rotatable member that is housed in the mounting arm and coupled to the stand. In this way, that stand can rotate freely via the pivot. The stand may include a base and two opposing sidewalls. In some embodiments, the sidewalls are disposed at obtuse angle relative to the base. The base may include a groove for receiving waste from a specimen. In some embodiments, the base includes an absorbent pad (e.g., within the groove or disposed on the base). The absorbent pad can be glued or otherwise attached to the base. In some embodiments, the stand can include an integrated heating device and heating sensors. For example, the stand may comprise a thermally conductive material such as a metal (e.g., aluminum) to distribute heat evenly to the animal. The heating element can be a removable insert that is received by the stand. The heating element may include a temperature sensor to maintain and facilitate precise temperature control. In some embodiments, an external heating element can be placed on the stand for heating.

In some embodiments, the mechanism includes a fluid delivery system. The fluid delivery system may be integrated into the apparatus for fluid (e.g., gas or liquid) delivery to the specimen. In some embodiments, the gas delivery system is integrated into the pivot of the animal stand to provide a low-profile fluid delivery system that does not occlude the imaging path. For example, the pivot can include an inlet to receive a fluid line to supply fluid to a specimen, and an outlet to remove fluid from the specimen. The inlet and outlet are provided to both supply and dispose of fluid thus preventing gas from escaping into the environment. The fluid delivery system provides a seamless way to route gas or other fluids without any risk of kinking or damaging delivery lines, while also improving hose management.

In some embodiments, the apparatus includes a sealing device. The sealing device can be removably attached to an end of the pivot. The sealing device is configured to provide a seal around the nose and mouth of a specimen to prevent gas from leaking into the environment while helping ensure a known concentration of fluid (e.g., anesthesia gas) is being delivered to the specimen. The sealing device may include an interior volume to receive the mouth and nose of the specimen. The entry to the sealing device may comprise a flexible material to fit snugly to the face of the specimen (e.g., a flexible material profiled to fit the face of a specimen). For example, the receiving end of the sealing device (e.g., a portion of the front face) may comprise a flexible material that conforms to the specimen. In some embodiments, the front face of the sealing device may comprise a shape memory material such as foam. The sealing device seals and directs the fluid delivery to the nose and mouth of the specimen while creating minimal additional bulk.

The sealing device may include at least two shafts extending from the front face of the sealing device. The at least two shafts extending from the front face of the sealing device may be positioned adjacent to the receiving end of the sealing device for receiving a portion of the face of a specimen. The shafts comprise a soft and flexible material, for example, a polymer-based material or foam. The shafts are configured to wipe along the face of the specimen as the sealing device is attached to the pivot. In this way, any external appendages (e.g., whiskers or eyelashes) can be pushed and restrained away from the face of the specimen (e.g., the eyes of the specimen). As such, external features of the specimen do not block the imaging path of an imaging device. For example, when imaging the eyes of a specimen, the shafts can pin down whiskers away from the eyes of the specimen.

In some embodiments, the mechanism includes integrated electronic circuitry that leads to the stand. The stand may include one or more sensor ports that is in communication with the electronic circuitry. In some embodiments, a connector may be coupled to at least one of the sensor ports. The connector is configured to provide one or more connection ports to attach an external device. For example, the one or more sensor ports may include connect ports for a blood oxygen monitor, a heart rate monitor, a blood pressure monitor, a respiration rate monitor, a blood oxygen monitor, or combinations thereof. In some embodiments, the connector is a magnetic connector.

The mechanism includes one or more restraints configured to retain a specimen on the stand. For example, the restraints may be configured to apply pressure between the specimen and the stand. In some embodiments, the restraints can be Velcro straps, a rubber strap, or a ratcheting mechanism. For example, the rubber strap may include a plurality of apertures and a pin. The pin can be inserted into one of the plurality of apertures to provide a snug fit of the specimen to the stand. In some embodiments, Velcro straps may be an external element (e.g., not coupled to the apparatus). In this way, the Velcro straps can be applied to desired region of the stand to restrain the specimen. The Velcro straps may extend around the mounting arm and the stand to provide a desired tension to the specimen.

The electrical ground may be grounding pads or grounding straps that make contact with the specimen. For example, the grounding pads may be in contact with paws of the specimen and the grounding straps may be in contact with a tail of the specimen. So, the electrical path can be established between the stand, the specimen, and a lens tip when an open circuit between the lens tip and the laser is closed.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of specific embodiments are presented for purposes of illustration and description. They are not intended to be exhaustive or to limit the described embodiments to the precise forms disclosed. It will be apparent to one of ordinary skill in the art that many modifications and variations are possible in view of the above teachings.

## Claims

1. An apparatus comprising:
a housing comprising a laser and a lens assembly;
a lens tip disposed on the lens assembly configured to contact an eye of a subject, wherein a portion of the lens tip is conductive; and
an open circuit between the lens tip and the laser;
wherein the laser is configured to emit when a conductive member is in contact with the lens tip to close the open circuit.

2. The apparatus of claim 1, wherein the lens tip comprises a contact region configured to contact the eye of the subject.

3. The apparatus of claim 2,
wherein the contact region is conductive, in particular electrically conductive, ionically conductive, or thermally conductive; and/or
wherein the contact region surrounds a portion of a lens of the lens assembly; and/or
wherein the contact region completely surrounds a lens of the lens assembly.

4. The apparatus of one of claims 1 to 3,
wherein a surface region of the lens tip is conductive; and/or
wherein the lens tip is conical; and/or
wherein the laser is in communication with a conductive portion of the lens tip.

5. The apparatus of one of claims 1 to 4, further comprising an electrical ground in contact with the subject.

6. The apparatus of claim 5,
wherein the conductive member comprises an eyeball of the subject;
wherein, in particular, the lens tip, the subject, and the electrical ground comprise an electrical path when the open circuit is closed, the apparatus in particular further comprising an amplifier circuit disposed between the laser and the lens tip, wherein the electrical path further comprises the amplifier circuit.

7. The apparatus of claim 5,
wherein the electrical ground comprises a probe attached to the subject; and/or
wherein the electrical ground comprises a clip; and/or
wherein the electrical ground comprises a conductive gel.

8. The apparatus of claim 5,
wherein the electrical ground comprises a mechanism for positioning the subject for imaging;
wherein, in particular, the mechanism comprises:
a base member comprising a first end and a second end;
an upright member disposed at or adjacent to the second end of the base member, wherein the upright member comprises a rotatable support;
an adjustable arm extending from the upright member, wherein the adjustable arm comprises:
a mounting arm coupled to the rotatable support on the upright member;
a pivot attached to a distal end of the mounting arm; and
a stand attached to the pivot and disposed over the mounting arm;
a first adjustment means disposed on the upright member and coupled to the adjustable arm via the rotatable support, wherein the first adjustment means is configured to adjust the yaw of the adjustable arm; and
a second adjustment means coupled to the upright member, wherein the second adjustment means is configured to move the upright member in a y-direction;
wherein the stand can be rotated away or toward the upright member via the pivot.

9. A method comprising:
providing an apparatus comprising:
a housing comprising a laser and a lens assembly;
a lens tip disposed on the lens assembly configured to contact an eye of a subject, wherein a portion of the lens tip is conductive; and
an open circuit between the lens tip and the laser;
wherein the laser is configured to emit when a conductive member is in contact with the lens tip to close the open circuit;
placing an electrical ground on the subject;
contacting the eye of the subject with the lens tip; and
emitting the laser in response to contacting the eye with the lens tip based on an electrical path established between the lens tip, the subject, and the electrical ground.

10. The method of claim 9, further comprising:
removing the eye of the subject from contacting the lens tip; and
terminating the laser in response to removing the eye from contacting the lens tip based on the electrical path being removed.

11. The method of claim 9, wherein the electrical ground comprises a mechanism for positioning the subject for imaging.

12. The method of claim 11, further comprising:
providing the mechanism for positioning the subject for imaging, the mechanism comprising:
a base member comprising a first end and a second end;
an upright member disposed at or adjacent to the second end of the base member, wherein the upright member comprises a rotatable support;
an adjustable arm extending from the upright member, wherein the adjustable arm comprises:
a mounting arm coupled to the rotatable support on the upright member;
a pivot attached to a distal end of the mounting arm, wherein the pivot comprises an inlet; and
a stand attached to the pivot and disposed over the mounting arm;
a first adjustment means disposed on the upright member and coupled to the adjustable arm via the rotatable support, wherein the first adjustment means is configured to adjust the yaw of the adjustable arm; and
a second adjustment means coupled to upright member, wherein the second adjustment means is configured to move the upright member in a y-direction;
wherein the stand can be rotated away or toward to the upright member via the pivot;
positioning the subject on the stand;
adjusting the position of the subject on the stand by exerting a force on one or more of the first adjustment means, the second adjustment means, or the pivot; and
emitting the laser with the subject on the stand.

13. A system for applying a laser, the system comprising:
an apparatus housing comprising a laser;
a camera disposed in the apparatus housing and comprising a lens assembly;
a lens tip disposed on the lens assembly configured to contact a conductive member a subject, wherein a portion of the lens tip is conductive; and
an electrical ground in contact with the subject, wherein the lens tip, the subject, and the electrical ground comprise an electrical path when the conductive member of the subject is in contact with the lens tip, and wherein the laser is configured to be emitted based on the electrical path.

14. The system of claim 13,
wherein the lens tip comprises a conical tip including a contact region that is conductive; and
wherein, in particular, the conical tip is thermally conductive, and/or electrically conductive, and/or ionically conductive.

15. The system of claim 13,
further comprising:
an open circuit between the lens tip and the laser;
wherein the laser is configured to be emitted when the conductive member is in contact with the lens tip to close the open circuit; and/or
further comprising:
a visual indicator configured to indicate a status of the electrical path.
